(19) Europäisches Patentamt
European Patent Office
Office européen des brevets

(11) Veröffentlichungsnummer: **0 275 522 B1**

(12) **EUROPÄISCHE PATENTSCHRIFT**

(45) Veröffentlichungstag der Patentschrift: **29.03.95**

(51) Int. Cl.$^{6}$: **C07D 401/12**, C07D 403/12, C07D 207/16, C07D 403/06, C09K 19/20

(21) Anmeldenummer: **87118930.4**

(22) Anmeldetag: **21.12.87**

(54) **Verwendung von optisch aktiven 1-Acylprolinestern als Dotierstoffe in Flüssigkristallmischungen und neue optisch aktive 1-Acyl-prolinester.**

(30) Priorität: **24.12.86 DE 3644522**

(43) Veröffentlichungstag der Anmeldung:
**27.07.88 Patentblatt 88/30**

(45) Bekanntmachung des Hinweises auf die Patenterteilung:
**29.03.95 Patentblatt 95/13**

(84) Benannte Vertragsstaaten:
**DE**

(56) Entgegenhaltungen:
**WO-A-86/02937**
**WO-A-87/02375**

(73) Patentinhaber: **HOECHST AKTIENGESELL-SCHAFT**

**D-65926 Frankfurt (DE)**

(72) Erfinder: **Günther, Dieter, Dr.**
**Nachtigallenweg 1A**
**D-6233 Kelkheim (Taunus) (DE)**
Erfinder: **Ohlendorf, Dieter, Dr.**
**Am Kühlen Grund 4**
**D-6237 Liederbach (DE)**
Erfinder: **Wingen, Rainer, Dr.**
**Rotkäppchenweg 10**
**D-6234 Hattersheim am Main (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Flüssigkristalle haben insbesondere im letzten Jahrzehnt Eingang in verschiedenste technische Gebiete gefunden, in denen elektrooptische und Anzeigevorrichtungs-Eigenschaften gefragt sind (z. B. in Uhren-, Taschenrechner- und Schreibmaschinenangzeigen). Diese Anzeigevorrichtungen beruhen auf den dielektrischen Ausrichtungseffekten in den nematischen, cholesterishen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technick wegen ihrer einzigartigen Eigenschaften sehr vielversprechende chemische Verbindungen sind, eher zu langsam. Dieser Nachteil macht sich insbesondere dann bemerkbar, wenn - was bei größeren Anzeigeelementflächen zwangsläufig der Fall ist - eine große Anzahl von Bildpunkten angesteuert werden muß, wodurch die Produktionskosten solcher, diese größeren Flächen enthaltenden Geräte wie Videogeräte, Oszillographen oder TV-, Radar-, EDV- oder Schreibautomaten-Bildschirme zu hoch wären.

Neben den nematischen und cholesterischen Flüssigkristallen haben seit einigen wenigen Jahren in zunehmendem Maße auch für Praxisanwendungen geneigt ("tilted")-smektische Flüssigkristall-Phasen an Bedeutung gewonnen. Wenn solchen geneigt-smektischen Phasen, insbesondere smektisch C ($S_c$) Phasen, geeignete Dotierstoffe zugesetzt werden, die eine sogenannte spontane Polarisation ($P_s$) zeigen oder in der Flüssigkristall-Phase induzieren, so können die Phasen in eine ferroelektrische Flüssigkristall-Phase umgewandelt werden (Benennung von $P_s$ in $nC \cdot cm^{-2}$).

Clark und Lagerwall konnten zeigen, daß die Verwendung solcher Flüssigkristallsysteme in sehr dünnen Zellen zu optoelektrischen Anzeigeelementen führt, die im Vergleich zu den herkömmlichen TN ("twisted nematic")-Zellen um bis zu etwa einem Faktor 1000 schnellere Schaltzeiten haben (vgl. z. B. Lagerwall et al. "Ferroelectric Liquid Crystals for Displays", SID Symposium, October Meeting 1985, San Diego, Ca., USA). Aufgrund dieser und anderer günstiger Eigenschaften, z. B. der bistabilen Schaltmöglichkeit und des nahezu blickwinkelunabhängigen Kontrasts, sind FLC grundsätzlich für die oben genannten Anwendungsgebiete, z. B. über eine Matrixansteuerung, gut geeignet.

Für die praktische Verwendung von ferroelektrischen Flüssigkristallen in optoelektrischen Anzeigen werden nun chiral smektische Phasen, z. B. $S_c$-Phasen, benötigt, die über einen großen Temperaturbereich stabil sind und hohe spontane Polarisation ($P_s$) und kurze Schaltzeiten ($\tau$) aufweisen. Es ist versucht worden, dieses Ziel mit flüssigkristallinen Verbindungen oder Mischungen von flüssigkristallinen Verbindungen zu erreichen, die selbst z. B. eine $S_c$-Phase haben und ferroelektrisches Verhalten zeigen (D. M. Walba, US-Patent 45 56 727). Dem sind jedoch Grenzen gesetzt. Größere Möglichkeiten bieten sich, wenn man flüssigkristalline Verbindungen oder Mischungen solcher Verbindungen, die eine smektische Phase aufweisen, aber nicht selbst optisch aktiv sind, mit einer optisch aktiven Verbindung dotiert, die selbst nicht flüssigkristallin zu sein braucht.

Es besteht daher ein Bedürfnis an Verbindungen, die bei Zusatz zu nicht optisch aktiven flüssigkristallinen Verbindungen oder Mischungen in geringer Menge eine hohe spontane Polarisation induzieren und zu kurzen Schaltzeiten führen.

Es wurde nun gefunden, daß optisch aktive 1-Acyl-prolinester als Dotierstoffe in derartigen Mischungen schon in geringen Mengen zu sehr kurzen Schaltzeiten führen.

Gegenstand der Erfindung ist daher die Verwendung von optisch aktiven 1-Acyl-prolinestern als Dotierstoffe in Flüssigkristallsystemen.
Gegenstand der Erfindung sind weiterhin Flüssigkristallsysteme, die optisch aktive 1-Acylprolinester enthalten sowie neue optisch aktive 1-Acyl-prolinester.

Aus WO 86/02937 ist bekannt, daß chirale Derivate natürlicher Aminosäuren als Dotierstoffe zur Erzeugung von $S_c$-Phasen geeignet sind. Bei den dort beschriebenen Verbindungen ist jedoch der Aminostickstoff nicht Bestandteil eines Ringsystems.

Die gemäß der Erfindung als Dotierstoffe in Flüssigkristallmischungen zu verwendenden 1-Acylprolinester entsprechen der allgemeinen Formel (I)

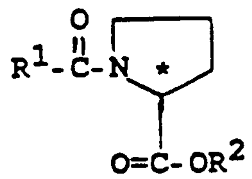

(I)

und sind ausgewählt aus der Gruppe bestehend aus

a) Verbindungen in denen ein Rest $R^1$ oder $R^2$

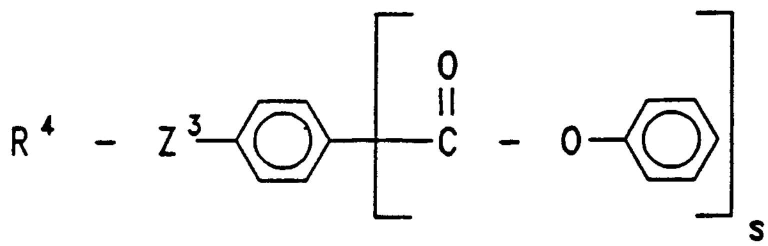

ist

wobei

| | |
|---|---|
| $Z^3$ | eine chemische Bindung oder ein Sauerstoffatom, |
| $R^4$ | eine geradkettige Alkylgruppe mit 6 bis 12 C-Atomen und |
| s | 1 bedeutet, |

und der andere Rest $R^1$ oder $R^2$ eine geradkettige oder verzweigte Alkylkette mit 1-12 C-Atomen, in der eine $CH_2$-Gruppe durch -O- ersetzt sein kann und die eine optische aktive Gruppe mit einem chiralen Baustein

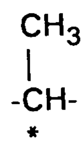

enthalten kann;

b) Verbindungen in denen ein Rest $R^1$ oder $R^2$ eine geradkettige oder verzweigte, gesättige, durch Cl substituierte Alkylgruppe mit 1 bis 12 C-Atomen ist und der andere Reste $R^1$ oder $R^2$

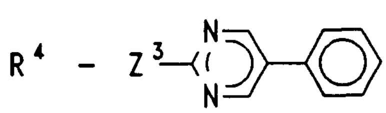 , 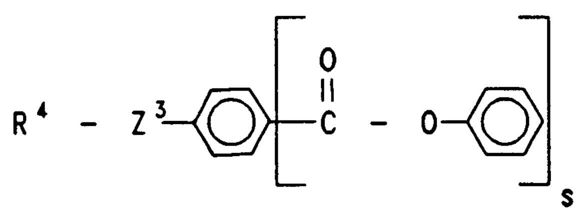

oder 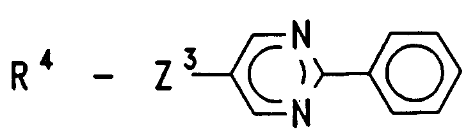

ist,

mit s = 1,
wobei $Z^3$ eine chemische Bindung oder ein Sauerstoffatom und $R^4$ eine geradkettige Alkylgruppe mit 6 bis 12 C-Atomen ist, und
c) die Verbindung in der

$R^1$ 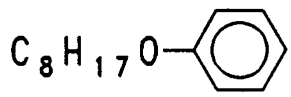

und $R^2$ n-$C_4H_9$ ist,
Weiterhin Gegenstand der Erfindung sind die Verbindungen
a) 1-[4-(4-Octyloxy-benzoyl]-oxybenzoyl]-(S)-(-)-prolin-n-butylester,
b) 1-[4-(4-Octyloxy-benzoyl)-oxybenzoyl]-(S)-(-)-prolinmethylester,
c) 1-[4-(4-Octyloxy-benzoyl)-oxybenzoyl]-(S)-(-)-prolin-n-hexylester,
d) 1-[4-(4-Octyloxy-benzoyl)-oxybenzoyl]-(S)-(-)-prolin-n-octylester,
e) 1-Propionyl-(S)-(-)-prolin-[4-(4-decyloxy-benzoyl]-oxyphenyl]-ester,
f) 1-Hexanoyl-(S)-(-)-prolin-[4-(4-decyloxy-benzoyl)-oxyphenyl]-ester,
g) 1-(2-Chlor-4-methyl-pentanoyl)-(S)-(-)-prolin-[4-(5-octylppyrimidin-2-yl)phenyl]-ester,
h) 1-[4-(4-Octyloxy-benzoyl)-oxybenzoyl]-(S)-(-)-prolin-iso-butylester,
i) 1-[(2S,3S)-2-Chlor-3-methyl-pentanoyl]-(S)-prolin-[4-(2-octylpyrimidin-5-yl)phenyl]-ester, und
j) 1-[4-(4-Octyloxy-benzoyloxy)benzoyl]-(S)-prolin-[(S)-2-chlor-propyl]-ester.

Die zur Einführung von mesogenen Resten eingesetzten Phenol-bzw. Benzoesäurederivate sind literaturbekannte Verbindungen.

Zur Herstellung der Verbindungen der Formel (I) wird am Stickstoff geschütztes Prolin in an sich bekannter Weise mit dem entsprechenden Phenol oder Alkohol verestert, und zwar in Gegenwart von Brönstedt- oder Lewis-Säuren, gegebenenfalls in Gegenwart wasserbindender Mittel oder unter Zuhilfenahme von Kondensationsmitteln wie N-N′-Carbonyldiimidazol oder Dicyclohexylcarbodiimid.

Die N-Acylierung erfolgt nach Abspaltung der Schutzgruppe mit den entsprechenden Carbonsäuren bzw. Carbonsäurechloriden in Gegenwart von Dicyclohexylcarbodiimid bzw. von organischen Basen wie Pyridin oder Triethylamin.

Die Flüssigkristallmischungen gemäß der Erfindung bilden Flüssigkristall-Phasen und enthalten mindestens eine optisch aktive Verbindung der allgemeinen Formel (I).

Unter dem Begriff "Flüssigkristallphase" sind nematische, cholesterische, smektische oder geneigt ("tilted")-smektische, insbesondere $S_c$-Phasen zu verstehen. Die Flüssigkristall-Mischungen bestehen aus 2 bis 20, vorzugsweise 2 bis 15 Komponenten, darunter mindestens eine der erfindungsgemäß beanspruchten chiralen Verbindungen.

Die anderen Bestandteile werden vorzugsweise ausgewählt aus den bekannten Verbindungen mit nematischen, cholesterischen und/oder smektischené, z. B. $S_A$-Phasen, und/oder geneigt smektischen Phasen; dazu gehören beispielsweise Schiffsche Basen, Biphenyle, Terphenyle, Phenylcyclohexane, Cyclohexylbiphenyle, N, S oder O-haltige Heterocyclen, z. B. Pyrimidine, Zimtsäureester, Cholesterinester, verschiedene überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d. h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Insbesondere enthält die Flüssigkristall-Mischung neben mindestens einer der erfindungsgemäß beanspruchten optisch aktiven Verbindungen eine Esterverbindung mit $S_c$-Phase, z. B. einen Alkoxybenzoesäurephenylester, oder eine biaromatische Verbindung mit einem stickstoffhaltigen Heterocyclus, z. B. ein Alkylpyrimindinyl-alkoxy-benzol.

Von der oder den erfindungsgemäßen Verbindung(en) enthalten die Flüssigkristallmischungen im allgemeinen 0,05 bis 70 Gew.%, insbesondere 0,1 bis 50 Gew.%.

Die erfindungsgemäßen Verbindungen sind insbesondere als Dotierstoffe für geneigt-smektische Flüssigkristallphasen geeignet, da sie diese in ferroelektrische Flüssigkristall-Phasen umwandeln; die Werte für die spontane Polarisation (Ps) liegen bei 10 Mol% Dotierung im Bereich von etwa 4 - 40 $nCb/cm^2$ und im Bereich von etwa 40 - 400 $nCb/cm^2$ extrapoliert auf die reine Verbindung.

Die Schaltzeiten der neuen Systeme liegen meistens deutlich unter 100µs.

Herstellungsbeispiele

Die Beispiele Nr. 1, 2, 4 - 10, 14, 16 - 24, 28, 29, 32 - 36, 39 und 41 - 44 sind Vergleichsbeispiele.

Beispiel 1:

1-(4-Palmityloxy)-benzoyl-(S)(-)prolin-methylester

10,9 g (30 mM) 4-Palmityloxy-benzoesäure werden mit 50 ml Thionylchlorid 1 Stunde am Rückfluß gerührt. Dann wird das überschüssige Thionylchlorid im Vakuum destilliert. Es verbleibt ein kristalliner Rückstand, der in eine Lösung von 5 g S-Prolinmethylester-chlorhydrat in 50 ml Pyridin bei Raumtemperatur eingetragen wird. Es wird 4 Stunden bei Raumtemperatur nachgerührt, auf 400 ml Eiswasser gegeben und mit konz. Salzsäure auf pH 3 - 4 gestellt. Das ausgefallene Produkt wird abgesaugt, mit Wasser gewaschen und bei 40 °C im Vakuum getrocknet. Das Rohprodukt wird sodann aus Methanol umkristallisiert. Man erhält 13,15 g (92 % d. Th.) der gewünschten Verbindung als farblose Kristalle vom Schmp.: 69 - 70 °C.
$[\alpha]_D^{20}$ :-33,6 ° (C = 1, $CH_2Cl_2$).

Beispiel 2:

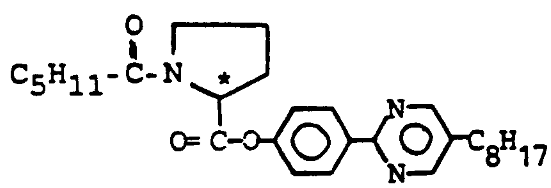

A. 12,5 g (50 mM) N-Carbobenzoxy-S-Prolin werden zussammen mit 14,2 g (50 mM) 2-(4-Hydroxyphenyl)-5-n-octylpyrimidin mit 0,75 g (5 mM) 4-Pyrrolidino-pyridin in 100 ml Methylenchlorid gelöst und auf 0 °C abgekühlt. Dann werden 11,1 g (54 mM) Dicyclohexylcarbodiimid zugegeben. Es wird 2 Stunden bei 0 ° - 5 °C gerührt, wobei der Dicyclohexylharnstoff sich abscheidet. Man läßt dann die Temperatur auf Raumtemperatur steigen un rührt noch weitere 5 Stunden. Man saugt vom Dicyclohexylharnstoff ab (11,35 g, 94 % d. Th.) und zieht das Lösungsmittel ab. Das dabei kristallisierende Rohprodukt wird aus 50 ml Methanol umkristallisiert. Man erhält 18,7 g (72 % d. Th.) der Verbindung

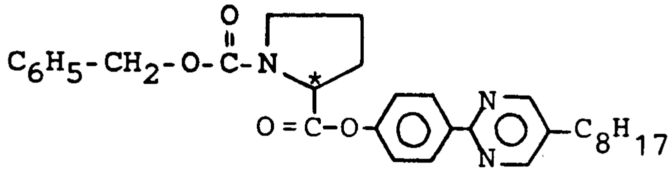

Schmp.: 97 - 98 °C $[\alpha]_D^{22}$ : -68,5 (C = 1, $CH_2Cl_2$)
B. 5,2 g (10 mM) der unter A dargestellten Verbindung werden in 6 ml Eisessig aufgeschlämmt und unter Rühren mit 6 ml HBr/Eisessig (30 %ig) versetzt. Die Kohlendioxidentwicklung ist nach 30 Minuten beendet. Man verdünnt mit 80 ml trockenem Diethylether, wobei das Hydrobromid des S-Prolinesters als Schmiere ausfällt. Nach 2 Stunden wird der Diethylether abdekantiert und mit weiteren 70 ml Diethylether erneut 3 Stunden gerührt, wobei das Hydrobromid erstarrt. Es wird schnell abgesaugt, mit Diethylether gewaschen und im Vakuum getrocknet und ohne Reinigung weiter umgesetzt.
C. 4,6 g (10 mM) des unter B hergestellten Hydrobromids werden in 25 ml Methylenchlorid mit 1,38 ml n-Hexansäurechlorid versetzt und auf 0 °C gekühlt. Dann werden 3,1 ml Triethylamin in 8 ml Methylenchlorid innerhalb von 5 Minuten zugetropft. Es wird noch 15 Minuten bei 0 ° - 5 °C und 1 Stunde bei Raumtemperatur gerührt. Das Lösungsmittel wird im Vakuum abdestilliert und der verbleibende Rückstand aus n-Hexan umkristallisiert. Man erhält 1,2 g (25 % d. Th.) der Titelverbindung.
Schmp.: 46 - 47 °C $[\alpha]_D^{22}$ : -54 ° (C = 1; $CH_2Cl_2$)

Beispiel 3:

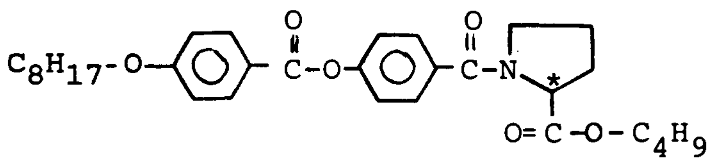

A. 5 g (20 mM) n-Carbobenzoxy-S-prolin werden mit 1,9 ml (20,8 mM) n-Butanol und 0,3 g (2 mM) 4-Pyrrolidino-pyridin in 40 ml Methylenchlorid gelöst und unter Rühren auf 0 °C abgekühlt. Danach gibt man 4,5 g (22 mM) Dicyclohexylcarbodiimid und hält die Temperatur weitere 2 Stunden bei 0 °C bis 5 °C.

Man läßt noch 24 Stunden bei Raumtemperatur stehen und saugt vom ausgeschiedenen Dicyclohexylharnstoff ab. Die Mutterlauge wird im Vakuum eingeengt, der Rückstand mit 50 ml Diisopropylether versetzt, nochmals vom Harnstoff abfiltriert und die Mutterlauge erneut eingeengt. Es verbleibt ein öliger Rückstand, der über 400 g Kieselgel mit Methylenchlorid/3 Vol % Methanol chromatographiert wird. Man erhält 4,1 g (67 % d. Th.) N-Carbobenzoxy-S-prolin-n-butylester als Öl.

$[\alpha]_D^{20}$ : -47,7 ° (C = 1, $CH_2Cl_2$)

B. 3,75 g(12.3 mM) des unter A dargestellten Z-S-Prolinesters werden mit 7 ml Eisessig und 7 ml HBr/Eisessig (33 %ig) bei Raumtemperatur versetzt und so lange gerührt, bis die Kohlendioxidentwicklung beendet ist (ca. 60 Minuten). Man versetzt mit 70 ml Diethylether. Es wird vom sich abscheidenden Öl abgetrennt und dieses im Hochvakuum bei Raumtemperatur getrocknet. Man erhält 2,9 g (93 % d. Th.) gelbliches Öl, das ohne weitere Reinigung weiter umgesetzt werden kann.

C. 4,55 g (12,3 mM) 4-(4-Octyloxy-benzoyloxy)-benzoesäure werden mit 20 ml Thionylchlorid 1 Stunde am Rückfluß gerührt. Die Lösung wird dann im Vakuum vom überschüssigen Thionylchlorid befreit (Kristallisation). Der Rückstand wird in 15 ml Methylenchlorid gelöst und zu einer auf 0 °C gekühlten Lösung von 2,9 g des unter B hergestellten Hydrobromids in 15 ml Methylenchlorid gegeben. Bei 0 ° bis 5 °C werden dann 3,8 ml (27 mM) Triethylamin in 8 ml Methylenchlorid innerhalb von 10 Minuten zugetropft. Man rühurt noch 15 Min. bei 0 °C und 1 Stunde bei Raumtemperatur, filtriert und engt die Mutterlauge im Vakuum zur Trockene ein. Man erhält einen halbkristallinen Rückstand, der über 220 g Kieselgel mit Methylenchlorid/10 Vol % Methanol als Laufmittel chromatographiert wird. Es verbleiben 4,5 Öl, das nach Dünnschichtchromatographie und H-NMR noch nicht rein ist. Es wird deshalb erneut über 200 g Kieselgel mit n-Hexan/20 Vol % n-Butanol chromatographiert. Man erhält 3,6 g (56 % d. Th. bez. auf einges. Carbonsäure) der Titelverbindung als farbloses Öl.

$[\alpha]_D^{21}$ : -31,1 ° (C = 1; $CH_2Cl_2$).

Die Verbindung zeigt folgendes Phasenverhalten: Kristallin -15 °C cholesterisch 14,5 °C isotrop.

Tabelle I

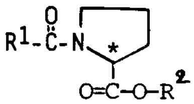

| Beispiel Nr. | $R^1$ | $R^2$ | K [°C]I | Drehung [Grad] ($C=1$, $CH_2Cl_2$) |
|---|---|---|---|---|
| 1 | $C_{16}H_{33}$-O- 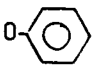 | $CH_3$ | 69-70 | $[\alpha]_D^{22}$: -33,6° |
| 2 | $C_5H_{11}$ | 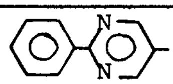 $C_8H_{17}$ | 97-98 | $[\alpha]_D^{22}$: -68,5° |
| 3 | $C_8H_{17}O$- 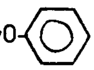 | n-$C_4H_9$ | Öl | $[\alpha]_D^{22}$: -31,1° |
| 4 | $C_{10}H_{21}$-O- 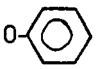 | $CH_3$ | · 49-50 · | $[\alpha]_D^{22}$: -40,7° |
| 5 | " | $C_2H_5$ | Öl isotrop bis -20° | $[\alpha]_D^{21}$: -30,7° |
| 6 | " | $C_4H_9$ | Öl isotrop bis -20° | $[\alpha]_D^{20}$: -28,8° |
| 7 | " | $C_6H_{13}$ | Öl isotrop bis -20° | $[\alpha]_D^{21}$: -26,6° |
| 8 | " | $C_8H_{17}$ | Öl | $[\alpha]_D^{22}$: -21,6° |
| 9 | $C_{16}H_{33}$-O- 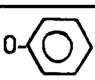 | $C_6H_{13}$ | Öl | $[\alpha]_D^{22}$: -21,3° |

Tabelle I (Fortsetzung)

| Beispiel Nr. | $R^1$ | $R^2$ | K [°C]I | Drehung [Grad] ($C=1$, $CH_2Cl_2$) |
|---|---|---|---|---|
| 10 | $C_{18}H_{37}O$-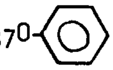 | $CH_3$ | · 63-66 · | $[\alpha]_D^{20}$: -25,5° |
| 11 | $C_8H_{17}O$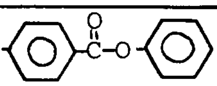 | $CH_3$ | · 60,9 · [9,2 Ch 28] | $[\alpha]_D^{20}$: -38,3° |
| 12 | " | $C_2H_5$ | Öl | 3955 $[\alpha]_D^{21}$: -29,5° |
| 13 | " | $C_6H_{13}$ | Öl ·-11 Ch 9,6· | $[\alpha]_D^{21}$: -29,4° |
| 14 | " | 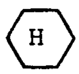 | Öl | $[\alpha]_D^{21}$: -29,2° |
| 15 | " | $C_8H_{17}$ | ·-2,5 Ch 15,1· | $[\alpha]_D^{21}$: -28,3° |
| 16 | $CH_3$ | 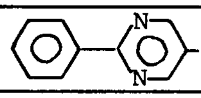 $C_8H_{17}$ | 48 | $[\alpha]_D^{22}$: -58,4° |
| 17 | $C_2H_5$ | " | · 63-64 · | $[\alpha]_D^{22}$: -63,3° |
| 18 | $C_3H_7$ | | 51-53 | $[\alpha]_D^{20}$: -56,5° |
| 19 | $C_7H_{15}$ | " | 68-69 | $[\alpha]_D^{22}$: -51,3° |

Tabelle I (Fortsetzung)

| Beispiel Nr. | $R^1$ | $R^2$ | K [°C]I | Drehung [Grad] (C=1, $CH_2Cl_2$) |
|---|---|---|---|---|
| 20 | $C_9H_{19}$ | 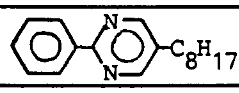 | 77-78 | $[\alpha]_D^{22}$: -49,7° |
| 21 | 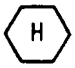 | " | 106-07 | $[\alpha]_D^{22}$: -50,6° |
| 22 | 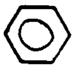 | " | 77-78 | $[\alpha]_D^{21}$: -50,4° |
| 23 | 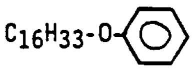 | " | 91-93 | $[\alpha]_D^{20}$: -42,8° |
| 24 | 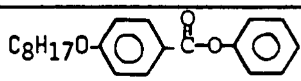 | " | 114 | $[\alpha]_D^{21}$: -28,4° |
| 25 | " | 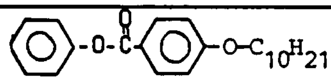 | 122-23 | $[\alpha]_D^{21}$: -24,2° |
| 26 | $C_2H_5$ | " | 61-62 | $[\alpha]_D^{22}$: -48,1° |
| 27 | $C_5H_{11}$ | " | 51-52 | $[\alpha]_D^{22}$: -44,0° |
| 28 | " | 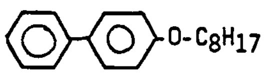 | 83-85 | $[\alpha]_D^{20}$: -53° |
| 29 | " | 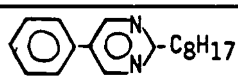 | 57-58 | $[\alpha]_D^{20}$: -55,5° |

Tabelle I (Fortsetzung)

| Beispiel Nr. | $R^1$ | $R^2$ | K [°C]I | Drehung [Grad] ($C=1$, $CH_2Cl_2$) |
|---|---|---|---|---|
| 30 | $CH_3CH-CH_2\overset{*}{C}H$<br>$CH_3$ Cl | 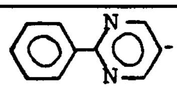 -$C_8H_{17}$ | 80-82 | $[\alpha]_D^{21}$: -63,4° |
| 31 | $C_8H_{17}O$-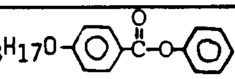 | $\overset{*}{C}H-CH_2-CH_3$<br>$CH_3$ | Öl | $[\alpha]_D^{21}$: -34,4° |
| 32 | $C_{10}H_{21}-O$-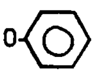 | 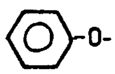 -O-$\overset{*}{C}H(CH_3)-COOC_2H_5$ | Öl | $[\alpha]^{21}$: -54,2° |
| 33 | " | 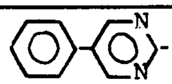 -$OC_8H_{17}$ | Öl | $[\alpha]_D^{21}$: -20,2° |
| 34 | $C_5H_{11}$ | " | 69-72 | $[\alpha]_D^{21}$: -52,0° |
| 35 | $C_{10}H_{21}-O$-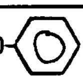 | 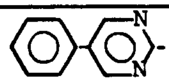 -S-$C_8H_{17}$ | 79-80 | $[\alpha]_D^{21}$: -19,8° |
| 36 | $C_5H_{11}$ | " | Öl | $[\alpha]_D^{21}$: -50,8° |

Tabelle I (Fortsetzung)

| Bei-spiel Nr. | $R^1$ | $R^2$ | K [°C]I | Drehung [Grad] (C=1, $CH_2Cl_2$) |
|---|---|---|---|---|
| 37 | $H_5C_2-\overset{*}{C}H-\overset{*}{C}H$ $CH_3$ Cl | 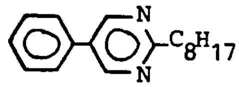 | 81-82 | $[\alpha]_D^{21}$: -63,4° |
| 38 | 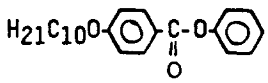 | $\overset{*}{C}H-C_2H_5$ $CH_3$ | Öl | $[\alpha]_D^{21}$: -31,8° |
| 39 | 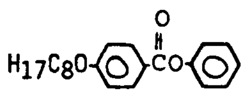 | $\overset{*}{C}HCO_2C_2H_5$ $CH_3$ | -10 | $[\alpha]_D^{21}$: -53,0° |
| 40 | " | $CH_2-\overset{*}{C}H-CH_3$ Cl | 16[a)] | $[\alpha]_D^{21}$: -22,7° |
| 41 | 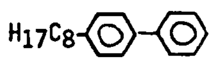 | $CH_3$ | 78-79 | $[\alpha]_D^{21}$: -34,3° |
| 42 | " | $C_8H_{17}$ | 28-29 | $[\alpha]_D^{21}$ -27,3 |
| 43 | 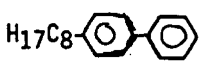 | $C_8H_{17}$ | 60-61 | $[\alpha]_D^{21}$: -29,1° |
| 44 | 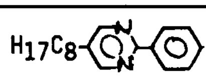 | $C_4H_9$ | Öl | $[\alpha]_D^{21}$: -28,3° |

a) Klärpunkt

Anwendungsbeispiele

Die Beispiele Nr. 1, 2, 4 - 10, 14, 16 - 24, 28, 29, 32 - 36, 39 und 41 - 44 sind Vergleichsbeispiele.

Zur Überprüfung der Wirksamkeit der vorstehend beschriebenen Verbindungen als Dotierstoffe in Flüssigkristall-Systemen werden diese in Konzentrationen von jeweils 10 Mol-% (oder in einzelnen Fällen von 5 und 17,5 Mol-%) mit dem Racemat der Verbindung

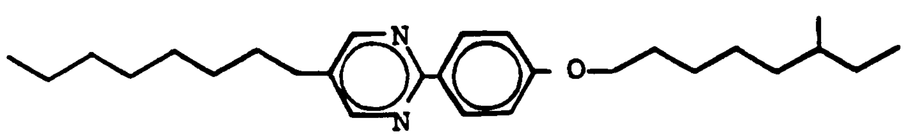

4-(5-Octyl-pyrimidin-2-yl)-1-(6-methyl-oct-1-oxy)benzol (Tabelle II) bzw. der Verbindung

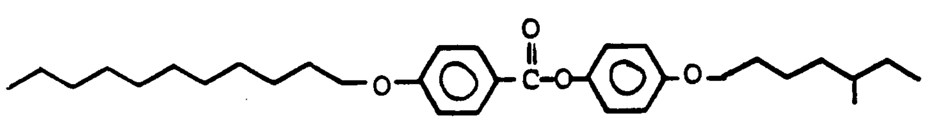

4-(4-Undecyloxy-phenyl-1-carbonyloxy)-1-(5-methylheptyloxy)-benzol (Tabelle (III) gemischt und jeweils die Werte für die spontane Polarisation ($P_s$ in $nC \cdot cm^{-2}$), für die Schaltzeit $\tau$ (in $\mu s$) und für den optischen Neigungswinkel der $S_c$-Phase $\theta$ (in °) der Mischung bestimmt. Die $P_s$-Werte werden nach der Methode von

Sawyer et al. (Phys. Rev. 35, 269 bis 273, 1930) gemessen, wobei eine spezielle Meßzelle [Skarp et al. in Ferroelectric Letters Vol. 06, 67 (1986)] verwendet wird, in der auch die $\tau$- und $\theta$-Werte bestimmt werden. Bei einer Zellenschichtdicke von ca. 2 µm wird durch Scherung eine einheitliche planare Orientierung der Flüsssigkristalle in der $S_c$-Phase erreicht [SSFLC-Technik, Clark et al., Appl. Phys. Lett. 36, 899 (1980)]. Zur Bestimmung von $\tau$ und $\theta$ wird die Meßzelle auf dem Drehtisch eines Polarisationsmikroskops zwischen gekreuztem Analysator und Polarisator befestigt. Durch Drehen der Meßzelle von maximalem zu minimalem Lichtdurchgang wird der optische Neigungswinkel bzw. Schaltwinkel, 2 $\theta$, bestimmt. Mit Hilfe einer Photodiode erfolgt die Bestimmung der Schaltzeit, $\tau$, indem die Anstiegszeit des Lichtsignals von 10 auf 90 % Signalhöhe gemessen wird. Tabelle II und III faßt die Ergebnisse für die Mischungen zusammen. Neben den Werten für $P_s$, $\tau$ und 2 $\theta$ ist der $S_c^*$ -Bereich der jeweiligen Mischung angegeben; die Werte in Klammern geben dabei die unterkühlbare untere Temperaturgrenze des $S_c^*$ -Bereichs an.

Tabelle II

jeweils bei 25°C

| Beisp. Nr. | Anteil der chiral. Verb. in mol% | $S_c^*$-Bereich der Mischung (°C) | Ps (nC/cm²) | $\tau$ (µs) | 2 $\theta$ (grad) |
|---|---|---|---|---|---|
| 2 | 10 | 13,5 [5] - 45 | 12 | 55 | 36 |
| 16 | 10 | 14,5 [2] - 43 | 6,5 | 45 | 40 |
| 17 | 10 | 13,5 [3] - 43 | 10,5 | 70 | 40 |
| 18 | 10 | 13,5 [5] - 45 | 11,5 | 50 | 33 |
| 19 | 10 | 15 [10] - 42 | 7,5 | 45 | 40 |
| 20 | 10 | 15 [5] - 43 | 4,5 | 80 | 35 |
| 21 | 10 | 13 [10] - 48 | 6 | 100 | 39 |
| 22 | 10 | 13 [4] - 48 | 13,1 | 40 | 42 |
| 30 | 5 | 20 - 45 | 19,5 | 60 | 34 |

Tabelle III

| Beisp. Nr. | Anteil der chiral. Verb. in mol% | $S_C^*$-Bereich der Mischung (°C) | jeweils bei 40°C Ps (nC/cm²) | τ (µs) | 2 θ (grad) |
|---|---|---|---|---|---|
| 1 | 10 | 32 - 50 | 5 | 50 | 32 |
| 3 | 10 | 21 - 59 | 31 | 35 | 62 |
| 4 | 10 | 30 [25] - 50 | 3,4 | 150 | 50 |
| 5 | 10 | 23,2 - 50 | 4 | 190 | 47 |
| 11 | 10 | 25 - 59 | 20 | 43 | 53 |
| 13 | 10 | 20 - 59 | 31 | 50 | 51 |
| 15 | 10 | 18 - 58 | 25 | 40 | 54 |
| 15 | 17,5 | 20 - 53 | 80 | 195 | 46 |
| 26 | 10 | 37 [28] - 58 | 20 | 30 | 57 |
| 27 | 10 | 38 [27] - 61 | 30 | 40 | 54 |
| 31 | 5 | 30 - 65 | 16 | 52 | 59 |
| 31 | 10 | 20 - 60 | 38 | 29 | 53 |
| 31 | 17,5 | 17,5 - 54 | 48 | 31 | 51 |
| 37 | 5 | 20 - 45 | 20 | 60 | 34 |
| 39 | 10 | 25 - 58 | 14 | 40 | 55 |
| 40 | 10 | 18 - 60 | 23 | 30 | 54 |
| 42 | 10 | 18 - 54 | 3 | 50 | 44 |
| 44 | 10 | 20 - 60 | 19 | 45 | 44 |

**Patentansprüche**

**1.** Verwendung von optisch aktiven 1-Acylprolinestern der Formel (I),

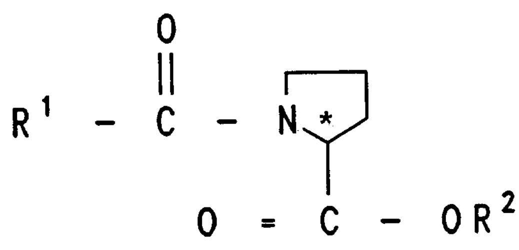

(I)

ausgewählt aus der Gruppe bestehend aus:

a) Verbindungen in denen ein Rest $R^1$ oder $R^2$

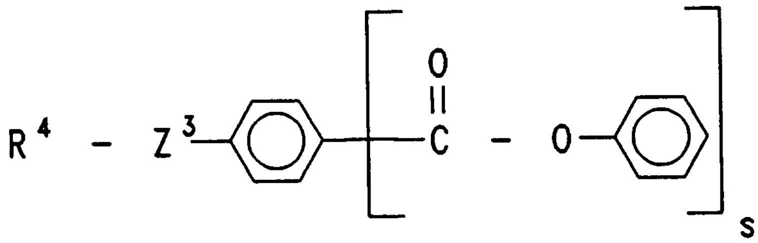

ist

wobei

- $Z^3$ eine chemische Bindung oder ein Sauerstoffatom,
- $R^4$ eine geradkettige Alkylgruppe mit 6 bis 12 C-Atomen und
- s 1 bedeutet,

und der andere Rest $R^1$ oder $R^2$ eine geradkettige oder verzweigte Alkylkette mit 1-12 C-Atomen, in der eine $CH_2$-Gruppe durch -O- ersetzt sein kann und die eine optische aktive Gruppe mit einem chiralen Baustein

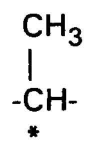

enthalten kann;

b) Verbindungen in denen ein Rest $R^1$ oder $R^2$ eine geradkettige oder verzweigte, gesättigte, durch Cl substituierte Alkylgruppe mit 1 bis 12 C-Atomen ist und der andere Reste $R^1$ oder $R^2$

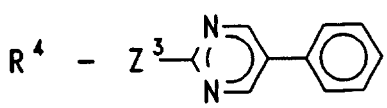 , 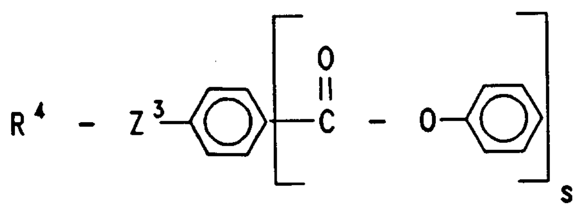

oder 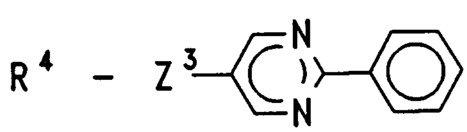

ist,

mit s = 1

wobei $Z^3$ eine chemische Bindung oder ein Sauerstoffatom und $R^4$ eine geradkettige Alkylgruppe mit 6 bis 12 C-Atomen ist, und

c) die Verbindung in der

$R^1$ 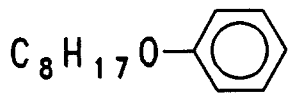

und $R^2$ $n\text{-}C_4H_9$ ist,
als Dotierstoffe in Flüssigkristallmischungen.

**2.** Verwendung von optisch-aktiven Prolinestern der Formel (I) in Anspruch 1, ausgewählt aus der Gruppe bestehend aus:
a) 1-[4-(4-Octyloxy-benzoyl)-oxybenzoyl]-(S)-(-)-prolin-n-butylester,
b) 1-[4-(4-Octyloxy-benzoyl)-oxybenzoyl]-(S)-(-)-prolinmethylester,
c) 1-[4-(4-Octyloxy-benzoyl)-oxybenzoyl]-(S)-(-)-prolin-n-hexylester,
d) 1-[4-(4-Octyloxy-benzoyl)-oxybenzoyl]-(S)-(-)-prolin-n-octylester,
e) 1-Propionyl-(S)-(-)-prolin-[4-(4-decyloxy-benzoyl]-oxyphenyl]-ester,
f) 1-Hexanoyl-(S)-(-)-prolin-[4-(4-decyloxy-benzoyl)-oxyphenyl]-ester,
g) 1-(2-Chlor-4-methyl-pentanoyl)-(S)-(-)-prolin-[4-(5-octylppyrimidin-2-yl)phenyl]-ester,
h) 1-[4-(4-Octyloxy-benzoyl)-oxybenzoyl]-(S)-(-)-prolin-iso-butylester,
i) 1-[(2S,3S)-2-Chlor-3-methyl-pentanoyl]-(S)-prolin-[4-(2-octylpyrimidin-5-yl)phenyl]-ester, und
j) 1-[4-(4-Octyloxy-benzoyloxy)benzoyl]-(S)-prolin-[(S)-2-chlor-propyl]-ester, als Dotierstoffe in Flüssigkristallmischungen.

**3.** Flüssigkristallmischungen, gekennzeichnet durch einen Gehalt an mindestens einer Verbindung nach Anspruch 1 und/oder 2.

**4.** Flüssigkristallmischung nach Anspruch 3, dadurch gekennzeichnet, daß sie 0,1 bis 50 Gew.-% an mindestens einer Verbindung nach den Ansprüchen 1 und/oder 2 enthält.

**5.** Flüssigkristallmischung nach Anspruch 3 und/oder 4, dadurch gekennzeichnet, daß sie ferroelektrisch ist.

**6.** 1-Acylprolinester, wie in Anspruch 1 und/oder 2 beschrieben.

**7.** Anzeigeelement, enthaltend eine Flüssigkristallmischung mit einem Gehalt an mindestens einem optisch aktiven 1-Acylprolinester nach den Ansprüchen 1 und/oder 2.

## Claims

**1.** Use of optically active 1-acylproline esters of the formula (I)

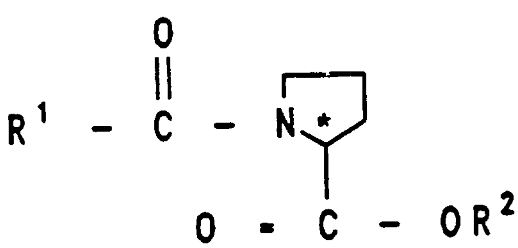 (I)

selected from the group consisting of:

a) compounds in which one radical $R^1$ or $R^2$ is

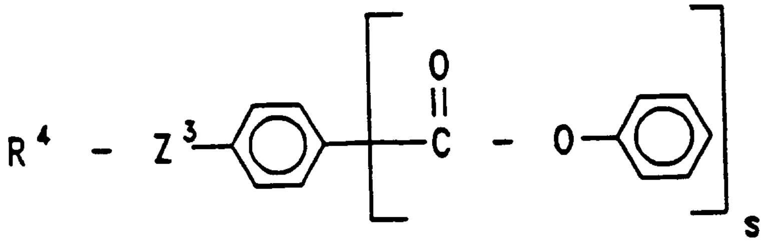

where

$Z^3$ is a chemical bond or an oxygen atom,

$R^4$ is a straight-chain alkyl group having 6 to 12 carbon atoms and

s is 1,

and the other radical $R^1$ or $R^2$ is a straight-chain or branched alkyl chain having 1-12 carbon atoms, in which one $CH_2$ group can be replaced by -O- and which can contain an optically active group containing a chiral unit

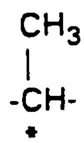 ;

b) compounds in which one radical $R^1$ or $R^2$ is a straight-chain or branched, saturated, Cl-substituted alkyl group having 1 to 12 carbon atoms and the other radical $R^1$ or $R^2$ is

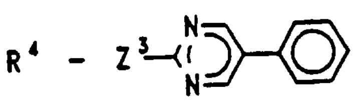 , 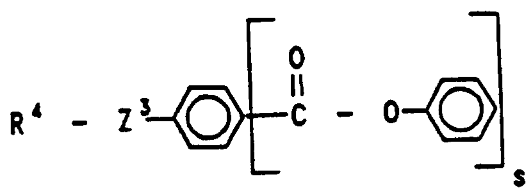

or 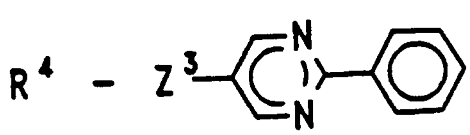

where s = 1,

$Z^3$ is a chemical bond or an oxygen atom and $R^4$ is a straight-chain alkyl group having 6 to 12 carbon atoms, and

c) the compound in which

$R^1$ is 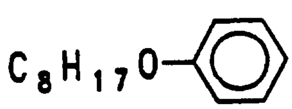

and $R^2$ is n-$C_4H_9$,

as dopes in liquid-crystal mixtures.

**2.** Use of optically active proline esters of the formula (I) in claim 1, selected from the group consisting of:
a) 1-[4-(4-octyloxybenzoyl)oxybenzoyl]-(S)-(-)-proline n-butyl ester,
b) 1-[4-(4-octyloxybenzoyl)oxybenzoyl]-(S)-(-)-proline methyl ester,
c) 1-[4-(4-octyloxybenzoyl)oxybenzoyl]-(S)-(-)-proline n-hexyl ester,
d) 1-[4-(4-octyloxybenzoyl)oxybenzoyl]-(S)-(-)-proline n-octyl ester,
e) 1-propionyl-(S)-(-)-proline 4-(4-decyloxybenzoyl)-oxyphenyl ester,
f) 1-hexanoyl-(S)-(-)-proline 4-(4-decyloxybenzoyl)-oxyphenyl ester,
g) 1-(2-chloro-4-methylpentanoyl)-(S)-(-)-proline 4-(5-octylpyrimidin-2-yl)phenyl ester,
h) 1-[4-(4-octyloxybenzoyl)oxybenzoyl]-(S)-(-)-proline isobutyl ester,
i) 1-[(2S,3S)-2-chloro-3-methylpentanoyl]-(S)-proline 4-(2-octylpyrimidin-5-yl)phenyl ester, and
j) 1-[4-(4-octyloxybenzoyloxy)benzoyl]-(S)-proline (S)-2-chloropropyl ester, as dopes in liquid-crystal mixtures.

**3.** A liquid-crystal mixture containing at least one compound as defined in claim 1 and/or 2.

**4.** A liquid-crystal mixture as claimed in claim 3, which contains from 0.1 to 50% by weight of at least one compound as defined in claims 1 and/or 2.

**5.** A liquid-crystal mixture as claimed in claim 3 and/or 4, which is ferroelectric.

**6.** A 1-acylproline ester as described in claim 1 and/or 2.

**7.** A display element containing a liquid-crystal mixture containing at least one optically active 1-acylproline ester as defined in claims 1 and/or 2.

**Revendications**

**1.** Utilisation d'esters optiquement actifs de la 1-acylproline de formule (I)

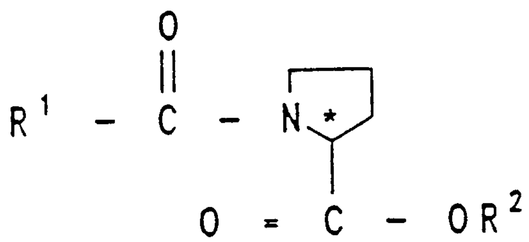

(I)

pris dans le groupe composé de :
a) composés dans lesquels un radical $R^1$ ou $R^2$ représente

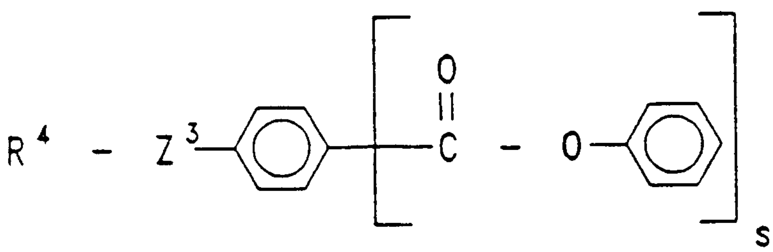

où
$Z^3$ représente une liaison chimique ou un atome d'oxygène
$R^4$ représente un groupe alkyle linéaire avec 6 à 12 atomes de carbone et
s signifie 1,

et les autres radicaux $R^1$ ou $R^2$ représentent une chaîne alkyle avec 1 à 12 atomes de carbone, dans laquelle un groupe $CH_2$ peut être remplacé par -O-et qui peut contenir un groupe optiquement actif avec un élément chiral

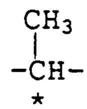

b) composés dans lesquels un radical $R^1$ ou $R^2$ est un groupe alkyle avec 1 à 12 atomes de C linéaire ou ramifié, saturé, substitué par Cl et l'autre radical $R^1$ ou $R^2$

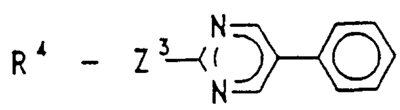
,
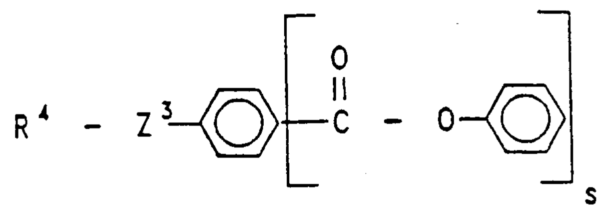

ou

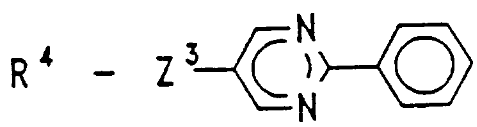

avec s = 1
où
$Z^3$ est une liaison chimique ou un atome d'oxygène et $R^4$ un groupe alkyle linéaire avec 6 à 12 atomes de C, et
c) le composé dans lequel

$R^1$ est 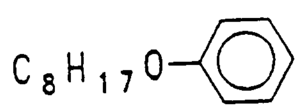

et $R^2$ est n-$C_4H_9$,
en tant qu'agents dopants dans des mélanges de cristaux liquides.

**2.** Utilisation d'esters de proline optiquement actifs de formule (I) dans la revendication 1, pris dans le groupe comportant les :
a) ester 1-[4-(4-octyloxy-benzoyl)-oxybenzoyl]-S-(-)-prolin-n-butylique
b) ester 1-[4-(4-octyloxy-benzoyl)-oxybenzoyl]-S-(-)-prolin-méthylique
c) ester 1-[4-(4-octyloxy-benzoyl)-oxybenzoyl]-S-(-)-prolin-n-hexylique
d) ester 1-[4-(4-octyloxy-benzoyl)-oxybenzoyl]-S-(-)-prolin-n-octylique
e) ester 1-propionyl-S-(-)-prolin-[4-(4-décyloxybenzoyl]-oxyphénylique]
f) ester 1-hexanoyl-S-(-)-prolin-[4-(4-décyloxybenzoyl]-oxyphénylique]
g) ester 1-(2-chloro-4-méthyl-pentanoyl-S-(-)-prolin-[4-(5-octylpyrimidin-2-yl)]-phénylique]
h) ester 1-[4-(4-octyloxy-benzoyl)-oxybenzoyl]-S-(-)-prolin-iso-butylique
i) ester 1-[(2S,3S)-2-chloro-3-méthyl-pentanoyl]-S-prolin-[4-(2-octylpyrimidin-5-yl)]-phénylique], et
j) ester 1-[4-(4-octyloxy-benzoyloxy)-benzoyl]-S-prolin-[(S)-2-chloro-propylique]
en tant qu'agents de dopage.

**3.** Mélanges de cristaux liquides caractérisés par une teneur en au moins un composé selon la revendication 1 et/ou 2.

4. Mélange de cristaux liquides selon la revendication 3, caractérisé en ce qu'il contient de 0,1 à 50% en poids en au moins un composé selon les revendications 1 et/ou 2.

5. Mélange de cristaux liquides selon la revendication 3 et/ou 4, caractérisé en ce qu'il est ferroélectrique.

6. Ester de la 1-acylproline comme décrit dans la revendication 1 et/ou 2.

7. Elément d'affichage contenant un mélange de cristaux liquides ayant une teneur en au moins un ester optiquement actif de la 1-acylproline selon les revendications 1 et/ou 2.